(19) Europäisches Patentamt
European Patent Office
Office européen des brevets

(11) EP 0 797 569 B1

(12) **EUROPEAN PATENT SPECIFICATION**

(45) Date of publication and mention of the grant of the patent:
**02.06.1999 Bulletin 1999/22**

(21) Application number: **95942114.0**

(22) Date of filing: **07.12.1995**

(51) Int Cl.[6]: **C07C 255/31**, A61K 7/46

(86) International application number:
**PCT/EP95/04887**

(87) International publication number:
**WO 96/18604 (20.06.1996 Gazette 1996/28)**

(54) **CYCLOHEX(EN)YL-PROPIONITRILES**

CYCLOHEX(EN)YL-PROPIONITRILE

CYCLOHEX(EN)YL-PROPIONITRILES

(84) Designated Contracting States:
**BE CH DE ES FR GB IT LI NL**

(30) Priority: **15.12.1994 EP 94309395**

(43) Date of publication of application:
**01.10.1997 Bulletin 1997/40**

(73) Proprietor: **QUEST INTERNATIONAL**
**1411 GP Naarden (NL)**

(72) Inventor: **ROSSITER, Karen, Jane**
**Great Chart Ashford Kent TN23 3BE (GB)**

(74) Representative: **Graham, John George et al**
**ICI Group Intellectual Property**
**P.O. Box 90**
**Wilton**
**Middlesbrough, Cleveland TS90 8JE (GB)**

(56) References cited:

| | |
|---|---|
| **GB-A- 1 402 950** | **US-A- 4 146 507** |
| **US-A- 4 156 690** | **US-A- 4 336 204** |

- **J. ORG. CHEM. (JOCEAH,00223263);85; VOL.50 (20); PP.3692-8, KYOTO UNIV.;FAC. SCI.; KYOTO; 606; JAPAN (JP), ONO N ET AL 'New synthetic methods. Conjugate addition of alkyl groups to electron deficient olefins with nitroalkanes as alkyl anion equivalents'**
- **J. ORG. CHEM. (JOCEAH);72; VOL.37 (12); PP.2035-9, FORDHAM UNIV.;DEP. CHEM.; NEW YORK; N. Y., MORICONI E J ET AL 'Effect of.alpha.-methyl substitution in the Beckmann and Schmidt rearrangement of 1-hydrindanones'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 797 569 B1

## Description

The present invention relates to certain β-cyclohexyl- and cyclohexenyl-propionitriles and acrylonitriles, to the use of these compounds as fragrance materials and to perfumes and perfumed products containing these compounds.

Many synthetic fragrance materials have been developed, especially in the last decades to substitute known perfume materials of natural origin. Nevertheless there is a constant need for new synthetic fragrance materials which are more stable than those previously developed and/or have additional or more delicate odour notes to further complete the fragrance palette from which the perfumer can chose in composing perfumes which are suitable e.g. for various aggressive environments.

Various aliphatic and aromatic nitriles are known in the art of perfumery. They were developed because of their good stability in agressive media and their general odour similarity with the corresponding aldehydes, many of which were already used in perfumery before the development of the nitriles. Various examples are described in S. Arctander, Perfume and Flavor Chemicals (Montclair, N.J., 1969), and by M. Grimberg, Riechstoffen, Aromen, Körperpflegemittel Nr. 4/64 103-109. Thus, Arctander monograph No. 764 describes 2,6,6-trimethyl-2-cyclohexenyl-acrylonitrile as having some odour resemblance to β-ionone, the corresponding methyl-ketone. Grimberg discloses the dihydro-analogue β-cyclogeranyl-acetonitrile and its α and γ-cyclogeranyl isomers to have an odour note resembling vetiver oil. Other examples of β-cyclohex(en)yl-substituted acrylonitriles are mentioned in US 4,336,204 which, however, all carry an isopropyl or isopropenyl side chain on the cyclohexyl ring. These compounds are said to display a wide variety of odour properties. Other fragrance materials carrying a cyclohexyl or cyclohexenyl as well as a nitrile group are disclosed in US 4,156,690 and in GB 1 402 950. These compounds from a chemical point of view are rather remote from β-cyclohexyl-propionitriles or acrylonitriles and again display a wide variety of odour properties.

This literature once more provides proof for what is common ground in the art of perfumery, i.e. that it is, as yet, impossible to predict organoleptic properties on the basis of chemical structure.

It has now been found that certain 2,4-dimethyl-cyclohex(en)yl-propionitriles and acrylonitriles and isomeric nitriles of the general formula below are valuable fragrance materials:

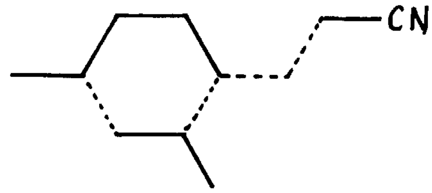

In this general formula the dotted lines may represent a double bond with the proviso that only one of the three connected dotted lines is a double bond. The compounds according to the invention generally have herbal-spicy and green odour notes. The completely saturated compound β-(2,4-dimethylcyclohexyl) propionitrile is primarily green and earthy in odour character. The compound in which only the single dotted line represents a double bond (β-(2,4-dimethylcyclohex-3-enyl)propionitrile) primarily has a spicy-cuminic odour character. The compounds in which both the single dotted line and one of the three connected dotted lines are double bonds have a caraway and green odour with a slight citrus note. Their odour character slightly changes to orris-like in the dry-out.

The compounds in which at least the single dotted line represents a double bond are preferred. Even more preferred are the compounds having two double bonds. Particularly preferred is 3-(2,4-dimethylcyclohex-3-enylidene)propionitrile.

The compounds according to the invention are not only novel as fragrance materials, they are also novel as such. They may be prepared according to methods known in the art for similar compounds, particularly by condensation of 2,4-dimethylcyclohex-3-enyl-carboxaldehyde (known in the art as Ligustral) or 2,4-dimethylcyclohexyl-carboxaldehyde with cyanoacetic acid, followed by decarboxylation. If Ligustral is used as the starting material a mixture of seven double bond isomers is obtained with respect to the position of the first double bond relative to the nitrile group and the attachment of the nitrile group containing side chain to the ring, i.e. cis α-β endo and exo, trans α-β endo and exo, cis β-γ, trans β-γ, γ-δ (endocyclic). The ratio between the isomers varied with the reaction conditions, e.g. increasing the time and temperature of the decarboxylation increases the amount of α-β isomers. Reducing the amount of alkaline catalyst favours the formation of β-γ and γ-δ isomers. Furthermore the isomer ratio in the mixture may be changed by acid-catalysed isomerization, particularly by p-toluene-sulphonic acid. Finally, the various isomers may be separated by fractional distillation or other suitable separation techniques. From a perfumery point of view these measures are not required since the reaction conditions may be easily chosen such that the most favoured β-γ product is obtained predominantly. Catalytic hydrogenation of the doubly unsaturated product obtained from Ligustral, e.g. using $PtO_2$ in methanol, produces a mixture of the completely saturated nitrile and the mono-unsaturated product wherein the single dotted line is the double bond. This mixture can again be separated by fractional distillation. Similar considerations apply to the product mixture obtained from dihydroligustral.

The nitriles according to the invention are powerful fragrance materials which may be used as such to impart, strengthen or improve the odour of a wide variety of products, or they may be used as a component of a perfume to contribute their odour character to the overall odour of such perfume. For the purposes of this invention a perfume is intended to mean a mixture of fragrance materials, if desired mixed with or dissolved in a suitable solvent or mixed with a solid substrate, which is used to impart a desired odour to the skin and/or any product for which an agreeable odour is indispensible or desirable. Examples of such products are: fabric washing powders, washing liquids, fabric softeners and other fabric care products; detergents and household cleaning, scouring and disinfection products; air fresheners, room sprays and pomanders; soaps, bath and shower gels, shampoos, hair conditioners and other personal cleansing products; cosmetics such as creams, ointments, toilet waters, preshave, aftershave, skin and other lotions, talcum powders, body deodorants and antiperspirants, etc.

Other fragrance materials which can be advantageously combined with the nitriles according to the invention in a perfume are, for example, natural products such as extracts, essential oils, absolutes, resinoids, resins, concretes etc., but also synthetic materials such as hydrocarbons, alcohols, aldehydes, ketones, ethers, acids, esters, acetals, ketals, nitriles, etc., including saturated and unsaturated compounds, aliphatic, carbocyclic and heterocyclic compounds.

Such fragrance materials are mentioned, for example, in S. Arctander, Perfume and Flavor Chemicals (Montclair, N.J., 1969), in S. Arctander, Perfume and Flavor Materials of Natural Origin (Elizabeth, N.J., 1960) and in "Flavor and Fragrance Materials - 1991", Allured Publishing Co. Wheaton, Ill. USA.

Examples of fragrance materials which can be used in combination with the nitriles according to the invention are: geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, styrallyl acetate, benzyl benzoate, amyl salicylate, dimethylbenzylcarbinol, trichloromethylphenylcarbinyl acetate, p-tert-butylcyclohexyl acetate, isononyl acetate, vetiveryl acetate, vetiverol, α-hexylcinnamaldehyde, 2-methyl-3-(p-tert-butylphenyl)propanal, 2-methyl-3-(p-isopropylphenyl)propanal, 3-(p-tert-butylphenyl)-propanal, 2,4-dimethylcyclohex-3-enyl-carboxaldehyde, tricyclodecenyl acetate, tricyclodecenyl propionate, 4-(4-hydroxy-4-methylpentyl)-3-cyclohexenecarboxaldehyde, 4-(4-methyl-3-pentenyl)-3-cyclohexenecarboxaldehyde, 4-acetoxy-3-pentyltetrahydropyran, 3-carboxymethyl-2-pentylcyclopentane , 2-n-heptylcyclopentanone, 3-methyl-2-pentyl-2-cyclopentenone, n-decanal, n-dodecanal, 9-decenol-1, phenoxyethyl isobutyrate, phenyl-acetaldehyde dimethylacetal, phenylacetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks tetralin musks isochroman musks macrocyclic ketones, macrolactone musks, ethylene brassylate.

Solvents which can be used for perfumes which contain compounds according to the invention are, for example: ethanol, isopropanol, diethyleneglycol monoethyl ether, dipropylene glycol, diethyl phthalate, triethyl citrate, isopropyl myristate, etc.

The quantities in which the nitriles according to the invention can be used in perfumes or in products to be perfumed may vary within wide limits and depend, inter alia, on the nature of the product, on the nature and the quantity of the other components of the perfume in which the compounds are used and on the olfactive effect desired. It is therefore only possible to specify wide limits, which, however, provide sufficient information for the specialist in the art to be able to use the ether-nitriles according to the invention for his specific purpose. In perfumes an amount of 0.001% by weight or more of the nitriles according to the invention will generally have a clearly perceptible olfactive effect. Preferably the amount is at least 0.01% by weight. The amount of the nitriles according to the invention present in products will generally be at least 1 ppm by weight, preferably at least 10 ppm.

The following examples are only intended to illustrate the preparation and use of the nitriles according to the invention, but the invention is not in any way limited thereto

## EXAMPLE 1

<u>Preparation of the doubly unsaturated nitriles</u>

A twenty litre reaction vessel fitted with a stirrer and a thermometer and equipped with a Dean Stark arrangement was charged with 1.85 kg cyanoacetic acid, 3.0 kg Ligustral, 0.05 kg ammonium acetate and 1.87 kg p-cymene. While stirring, the mixture was heated to reflux and within three hours about 0.4 1 of water was removed azeotropically. The co-distilled cymene was returned to the reaction mixture which was again stirred and heated for another 3 hours at about 150°C pot-temp. After cooling the reaction mixture to well below 100°C the reaction mixture was washed first with 1 1 of a 5% salt solution followed by 1 1 of a 5% sodium carbonate solution. p-Cymene was removed by distillation under reduced pressure. Final traces of p-cymene were removed by azeotropical distillation under reduced pressure after 11 of water had been added to the reaction mixture. The crude nitriles mixture thus was further purified by distillation under reduced pressure, yielding 3.5 kg (78.8.% of theoretical yield) double unsaturated nitriles mixture. The isomer ratio was as set out below:

| | |
|---|---|
| Cis α-β (endo + exo) | 0.2% |
| Trans α-β (endo + exo) | 2.3% |
| β-γ (cis + trans) | 91.1% |
| γ-δ | 6.4% |

The condensation reaction was also carried out in refluxing toluene as the solvent using 250g of ammonium acetate instead of 54 g. After removal of the water the temperature of the reaction vessel was raised to about 160°C for one hour to effect the decarboxylation. The reaction mixture was further worked up as outlined above to yield 2.36 kg (53% of theoretical yield). The isomer ratio was as given below:

| | |
|---|---|
| Cis α-β (endo + exo) | 6.6% |
| Trans α-β (endo + exo) | 11.9% |
| β-γ (cis + trans) | 82.7% |
| γ-δ | 1.8% |

**EXAMPLE 2**

A perfume of the citrus-floral type for use in bath foam was prepared according to the following recipe:

| | % w/w |
|---|---|
| Nerolin | 0.5 |
| Galbanum resinoid (Q) | 1 |
| Coumarin | 2 |
| Lemon Oil | 2 |
| Grapefruit Oil | 3 |
| Traseolide (Q) | 3 |
| Orange Oil Florida | 3.5 |
| Lavandin abrialis | 9 |
| Bergamot Oil synthetic (Q) | 10 |
| Linalool | 14 |
| Linalyl acetate | 20 |
| p-Tert.butyl-dihydrocinnamic aldehyde | 29.5 |
| Product of EXAMPLE 1 | 2.5 |
| Total | 100 |

**Claims**

1. Nitriles according to the formula:

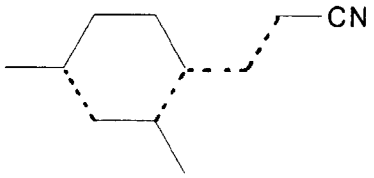

wherein the dotted lines may represent a double bond with the proviso that only one of the three connected dotted lines is a double bond.

2. Nitriles according to claim 1, characterized in that at least the single dotted line is a double bond.

3. Nitriles according to claim 2, characterized in that the single dotted line and one of the three connected dotted lines are double bonds.

4. A nitrile according to claim 1 which is 3-(2,4-dimethylcyclohex-3-enylidene)propionitrile.

5. Perfumes characterized in that they contain at least one nitrile according to any one of claims 1 to 4.

6. Perfumed products characterized in that they contain a nitrile according any one of claims 1 to 4.

7. Process for the preparation of a fragrance material containing at least one nitrile according to any one of claims 1 to 4 characterized in that 2,4-dimethylcyclohex-3-enyl-carboxaldehyde or 2,4-dimethylcyclohexyl-carboxaldehyde is condensed with cyanoacetic acid, followed by decarboxylation.

8. Process according to claim 7 characterized in that the condensation product is subjected to acid-catalysed isomerization.

9. Process according to claim 8 characterized in that the isomerization catalyst is p-toluene-sulphonic acid.

10. Process according to any one of claims 7 to 9 characterized in that the reaction product is subjected to catalytic hydrogenation.

11. Process according to any one of claims 7 to 10 characterized in that the starting material is 2,4-dimethylcyclohex-3-enyl-carboxaldehyde.

**Patentansprüche**

1. Nitrile nach der Formel:

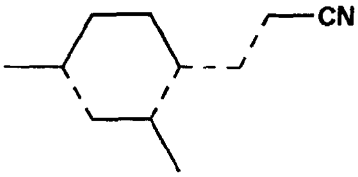

worin die gestrichelten Linien eine Doppelbindung darstellen können, mit der Maßgabe, daß nur eine der drei verbundenen gestrichelten Linien eine Doppelbindung ist.

2. Nitrile nach Anspruch 1, dadurch gekennzeichnet, daß zumindest die alleinstehende gestrichelte Linie eine Doppelbindung ist.

3. Nitrile nach Anspruch 2, dadurch gekennzeichnet, daß die alleinstehende gestrichelte Linie und eine der drei verbundenen gestrichelten Linien Doppelbindungen sind.

4. Nitril nach Anspruch 1, welches 3-(2,4-Dimethylcyclohex-3-enyliden)propionitril ist.

5. Parfüme, dadurch gekennzeichnet, daß sie zumindest ein Nitril nach einem der Ansprüche 1 bis 4 enthalten.

6. Parfümierte Produkte, dadurch gekennzeichnet, daß sie ein Nitril nach irgendeinem der Ansprüche 1 bis 4 enthalten.

7. Verfahren zur Herstellung eines Aromastoffs, welcher zumindest ein Nitril nach irgendeinem der Ansprüche 1 bis 4 enthält, dadurch gekennzeichnet, daß 2,4-Dimethylcyclohex-3-enylcarboxaldehyd oder 2,4-Dimethylcyclohexylcarboxaldehyd mit Cyanoessigsäure kondensiert wird, gefolgt von einer Decarboxylierung.

8. Verfahren nach Anspruch 7, dadurch gekennzeichnet, daß das Kondensationsprodukt einer säurekatalysierten Isomerisierung unterzogen wird.

9. Verfahren nach Anspruch 8, dadurch gekennzeichnet, daß der Isomerisierungskatalysator p-Toluolsulfonsäure ist.

10. Verfahren nach irgendeinem der Ansprüche 7 bis 9, dadurch gekennzeichnet, daß das Reaktionsprodukt einer katalytischen Hydrierung unterzogen wird.

11. Verfahren nach irgendeinem der Ansprüche 7 bis 10, dadurch gekennzeichnet, daß das Ausgangsmaterial 2,4-Dimethylcyclohex-3-enylcarboxaldehyd ist.

**Revendications**

1. Nitriles selon la formule :

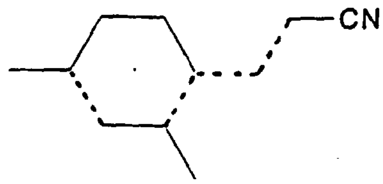

dans laquelle la ligne pointillée peut représenter une double liaison à la condition que seulement l'une des trois lignes pointillées reliées entre elles représente une double liaison

2. Nitriles selon la revendication 1, caractérisés en ce qu'au moins la ligne pointillée unique représente une double liaison.

3. Nitriles selon la revendication 2, caractérisés en ce que la ligne pointillée unique et l'une des trois lignes pointillées reliées entre elles représentent des doubles liaisons.

4. Nitrile selon la revendication 1, qui est le 3-(2,4-diméthylcyclohex-3-énylidène)propionitrile.

5. Parfums caractérisés en ce qu'ils contiennent au moins un nitrile selon l'une quelconque des revendications 1 à 4.

6. Produits parfumés caractérisés en ce qu'ils contiennent un nitrile selon l'une quelconque des revendications 1 à 4.

7. Procédé pour la préparation d'un produit odorant contenant au moins un nitrile selon l'une quelconque des revendications 1 à 4, caractérisé en ce que le 2,4-diméthylcyclohex-3-ényl-carboxaldéhyde ou le 2,4-diméthylcyclohexyl-carboxaldéhyde est condensé avec l'acide cyanoacétique, laquelle opération est suivie d'une décarboxylation.

8. Procédé selon la revendication 7, caractérisé en ce que le produit de la condensation est soumis à une isomérisation catalysée par un acide.

9. Procédé selon la revendication 8, caractérisé en ce que le catalyseur d'isomérisation est l'acide p-toluènesulfonique.

**10.** Procédé selon l'une quelconque des revendications 7 à 9, caractérisé en ce que le produit de la réaction est soumis à une hydrogénation catalytique.

**11.** Procédé selon l'une quelconque des revendications 7 à 10, caractérisé en ce que le produit de départ est le 2,4-diméthylcyclohex-3-ényl-carboxaldéhyde.